# FASCICULE DE BREVET EUROPEEN

(11) **EP 0 658 124 B1**
(45) Date de publication et mention de la délivrance du brevet: **04.06.1997**
(21) Numéro de dépôt: 93918811.6
(22) Date de dépôt: 07.09.1993
(51) Int. Cl.: A61L 15/12, A61L 15/14, C09J 7/04, C08L 75/06, C08L 67/04

(54) **COMBINAISON SOUS FORME COMPOSITE DE MATIERES, SOUPLES OU RIGIDES, FORMABLES ET ADHESIVES SOUS L'EFFET D'UNE TEMPERATURE INFERIEURE A 90 oC**
FLEXIBLE ODER STARRE, FORMBARE UND ADHÄSIVE VERBUNDWERKSTOFFE MIT EINER VERARBEITUNGSTEMPERATUR UNTER 90 oC
COMBINATIONS IN COMPOSITE FORM OF FLEXIBLE OR RIGID MATERIALS WHICH ARE FORMABLE AND ADHESIVE AT TEMPERATURES BELOW 90 oC

(30) Priorité: 07.09.1992 BE 9200787
(43) Date de publication de la demande: 21.06.1995
(73) Titulaire: L'UNIVERSITE DE LIEGE, B-4000 Liège (BE); Liegeois, Jean-Marie, B-4654 Charneux-Herve (BE)
(72) Inventeur: LIEGEOIS, Jean-Marie, B-4654 Charneux/Herve (BE)
(74) Mandataire: Van Malderen, Joelle
(86) Numéro de dépôt international: BE9300057
(87) Numéro de publication internationale: WO9405339

(56) Documents cités:
- EP-A- 0 413 339
- DE-A- 3 605 192
- FR-A- 2 376 170
- US-A- 4 105 025
- US-A- 4 316 457
- US-A- 4 326 509
- DATABASE WPI Week 8713, 27 Mai 1987 Derwent Publications Ltd., London, GB; AN 87-089384 & JP,A,62 038 167 (KURODA) 19 Février 1987

## Description

La présente invention se rapporte à de nouvelles combinaisons composites en feuilles, plaques, bandes ou stratifiés thermomalléables et thermoadhésifs sur eux-mêmes, souples ou rigides et à prise rapide ainsi qu'à des procédés de préparation de ces combinaisons composites.

A côté des applications usuelles des matières plastiques, il y a divers domaines d'application potentielle où il est demandé de réaliser chaque fois une exécution unique d'un objet ou d'un assemblage dans une matière que l'on désire pouvoir mouler, former, modeler, de préférence à la main et par un traitement préalable simple tel qu'un réchauffage à une température facilement accessible.

Par le traitement préparatoire à leur utilisation, ces matières doivent donc acquérir une formabilité adéquate ainsi qu'un pouvoir adhésif sur elles-mêmes. Le traitement préparatoire envisagé ici se résume à une mise en température qui sera précisée plus loin, à l'exclusion d'usage de tout solvant ou adhésif externe.

L'application visée peut porter sur un objet ou une réalisation aussi rigide que possible tout comme on peut rechercher dans l'application, une certaine souplesse du matériau à pouvoir choisir suivant les cas.

De telles applications sont souvent recherchées par des personnes n'ayant pas nécessairement une habilité manuelle poussée, qui veulent excercer elles-mêmes sans moyens techniques conséquents et en réalisant le moulage, le modelage ou l'assemblage principalement à la main avec l'aide éventuelle de quelques instruments simples tels que ciseaux, pinces et supports. Sans doute, les applications visées ici peuvent être pratiquées par des professionels également exerçant dans un environnement industriel mais ce n'est pas requis.

Il résulte que ces applications de formage et laminage doivent pouvoir être réalisées à partir d'une température de traitement préparatoire qui reste accessible sans risque de brûlures ou d'autre inconvénient pouvant causer une maladresse ou un accident.

La matière employée doit perdre son caractère adhésif une fois revenue à température ambiante et conserver aussi la forme qui lui a été donnée de même qu'elle doit être apte à résister aux contraintes mécaniques auxquelles elle pourrait être exposée et ne pas perdre sa forme jusqu'à une température la plus élevée possible dictée par l'application.

Un domaine de température pour le traitement préparatoire allant de 40 à 80°C paraît convenable et en tout cas ne dépassant pas 100°C de sorte que cette préparation peut se faire aisément et relativement rapidement en utilisant de l'eau chaude par exemple. Il va de soi que des sources de chaleur alternatives peuvent être utilisées également comme l'étuve, le four, le pistolet chauffant ou le champ de micro-ondes.

Vu les applications visées, il est aussi recommandé que ces matières soient inertes et ne relarguent pas de substances toxiques, irritantes ou d'un autre caractère nocif de sorte que l'applicateur puisse les utiliser sans moyen de protection particulier.

Ces produits doivent aussi être stables pour une longue durée sans être sujet à un veillissement chimique ni de préférence également à tout vieillissement physique.

Il ne faut pas non plus devoir recourir pour ce faire à un mode d'emballage sophistiqué tel que des pochettes hermétiques qui risquent de se déchirer ou qui compromettent l'usage de la matière une fois la pochette ouverte comme ce peut être le cas dans l'application du brevet européen 0 413 399 A1.

Il existe déjà quelques domaines d'application du type de produits visés par la présente invention où on utilise des composites formables et thermoadhésifs à partir d'une température de traitement allant de 70 à 80°C environ et qui sont essentiellement confinés dans des usages où on recherche une grande rigidité. Il s'agit principalement de la physiothérapie où l'on réalise ainsi déjà des attelles, orthèses et autres moyens de support rigides, de l'orthopédie intéressée par des bandages de contention cylindrique dits thermoplastiques ou par des prothèses externes du même matériau, mais aussi du domaine de la décoration, où des décors de théâtre, des masques et objets divers essentiellement en relief sont ainsi façonnés par laminage de 2 ou plusieurs couches.

Pour obtenir l'effet de formabilité et de thermoadhésivité dans le domaine de température souhaité, on dispose principalement de polymères linéaires d'estercyclique caractérisés par les fonctions "COO" séparées par des radicaux méthylènes comprenant de 2 à 7 carbones dont le principal représentant disponible est le polymère du 2-oxepanone mieux connu sous l'appelation polycaprolactone (PCL). Le brevet des Etats-Unis d'Amérique 3,692,023 suggère l'emploi de ce polymère pour l'obtention de matériel de contention à partir d'un substrat enduit du polymère et le brevet français 2,376,170 conduit au même résultat par un procédé seulement perfectionné. Par ailleurs, le brevet des Etats-Unis d'Amérique 4,273,115 préconise un tricot Raschel particulier partiellement imprégné de ce polymère PCL de poids moléculaire 40 000 environ comme matériel de contention cylindrique. Enfin, dans le brevet des Etats-Unis d'Amérique 4,316,457 on a proposé la préparation d'un polyuréthanne thermoplastique linéaire d'un poids moléculaire de 40 000 à 45 000, synthétisé en deux étapes où un prépolymère à deux moles d'un diisocyanate et une mole d'un polyester diol est additionné d'une mole du polyester diol avant imprégnation d'un tricot Raschel pour donner l'extension de chaîne en achevant la polymérisation sur celui-ci en même temps que le solvant s'évapore.

Tels que décrits, ces polyesters deviennent fluides à la température de fusion et ont une adhésivité excessive sur tout objet auquel il viennent en contact à l'état fondu. Cette adhésivité à l'état fondu qui entraîne un dépôt de matière est néanmoins perdue après durcissement par refroidissement. C'est pourquoi, l'utilisation de ces polyesters en orthopédie a nécessité diverses adaptations et on peut remarquer que les polyesters de condensation se comportent de la même manière.

Dans le brevet des Etats-Unis d'Amérique 3,692,023, on utilise une couche de base souple pour éviter que le polymère adhère à la peau, aux poils et aux cheveux qu'il recouvre lorsqu'on l'y applique. Dans le même brevet, on envisage de presser le même polymère en feuille, sur un support ou en sandwich fait d'un tissu, tricoté, tissé ou non tissé dont le rôle est d'empêcher l'écoulement de la matière à l'état fondu. Dans les brevets des Etats-Unis d'Amérique 4,273,115 et 4,316,457 relatifs aux bandages orthopédiques, on utilise une pellicule de séparation en polyéthylène qui est bobinée en même temps que le bandage pour éviter la prise en masse de celui-ci au moment de son application. Le brevet des Etats-Unis d'Amérique 4,143,655 préconise un autre type de séparateur à passages multiples tandis que le brevet des Etats-Unis d'Amérique 4,454,873 permet de supprimer l'effet gênant d'un séparateur par application d'un film polymère antiadhérant et hydrosoluble sur le matériau du bandage lui-même. Cette dernière solution a cependant l'inconvénient de contaminer progressivement l'eau du bain-marie de préparation et de réduire considérablement la résistance interlaminaire entre les différents plis d'une contention cylindrique notamment.

Il y a aussi des adaptations physiques internes au matériau qui contient ces polyesters. On a pu remarquer en effet que le type de tricot Raschel à brins volumineux faits de fibres coupées décrit dans les brevets des Etats-Unis d'Amérique 4,273,115 et 4,316,457 est absolument nécessaire pour supprimer l'effet de la trop grande fluidité des polycaprolactones et des polyuréthannes linéaires s'y rapportant, appliqués dans un bandage orthopédique par exemple. Ces brins très ouverts ont une densité apparente très faible et offrent un volume entre fibres d'environ 80 pour cent. Le brevet des Etats-Unis d'Amérique 4,273,115 précise d'ailleurs que les brins de fibres ne peuvent être que partiellement imprégnés du polymère. La résistance mécanique de ces brins, nécessairement peu noués et partiellement imprégnés, est dès lors assez faible, ce qui est constaté en particulier sur le produit fini imprégné de résine et que l'on peut facilement déchirer à la main. Les éléments de brin étant ainsi enrobés de polymère, l'ensemble s'oppose à l'écoulement fluide du composant thermoadhésif. Celui-ci reste néanmoins très déformable d'une manière plastique, et l'usage d'une bobine de ce matériau traité à température, conduit rapidement à une masse compacte qu'il devient très difficile de dérouler si l'on n'a pas au préalable, posé un film intercalaire de polyéthylène qui est bobiné en même temps que le bandage proprement dit.

Les nouveaux objectifs actuellement visés dans les applications basées sur ce savoir-faire ne sont pas satisfaits par ces polyesters aliphatiques ou ces polyuréthannes linéaires qui s'y rapportent s'ils sont appliqués isolément. En effet, si on essaie d'appliquer les polymères décrits dans les brevets des Etats-Unis d'Amérique 4,273,115 et 4,316,457 sur un tissu ou un tricot à mailles ouvertes notamment, et de haute ténacité ce qui n'est possible qu'en choisissant des brins plus serrés, mieux noués et faits de fibres continues, on est obligé, pour obtenir le même poids spécifique de résine thermoadhésive, d'enrober davantage l'extérieur du brin duquel, la résine qui n'est plus retenue par les fibres peut se déplacer de manière indésirable et être transférée sur les outils ou les mains de l'opérateur pendant l'application.

En outre, les tricots à brins volumineux tels que recommandés dans ces brevets sont d'une souplesse qui ne permet pas de les manipuler en largeur inférieure à un mètre environ. Dans ces conditions déjà, il y a rapprochement des brins de chaîne sous l'effet de la traction et du poids lorsqu'on imprègne par les procédés verticaux usuels également décrits dans ces mêmes brevets.Des bandages de largeurs usuelles sont donc obtenus dans ce cas, par découpe longitudinale après imprégnation du textile en grande largeur et séchage. Cette découpe résulte sur des aspérités à l'endroit des brins transversaux qui ont été tranchés. Il y a un intérêt évident d'obtenir un bandage en largeur utile et bordé de lisières uniformes exemptes d'aspérités. Toutefois, dans la configuration textile nécessaire aux polymères des brevets cités, les bandes de petites largeurs ne sont pas assez soutenues et le produit fini se retrouve sensiblement plus étroit qu'au point de départ. On observe d'ailleurs parallèlement, avec ce type de tricot volumineux fait de brins à fibres coupées, un allongement dans l'autre direction qui est rendu au moment du chauffage préparatoire à l'utilisation. Les substrats textiles nécessaires pour les polymères du savoir-faire antérieur ne permettent donc pas d'obtenir un produit stable dimensionnellement, il est nécessaire de préparer les bandes par découpe après dépôt du polymère. Si par ailleurs, on est intéressé par ces tricots imprégnés non pas dans l'optique d'un bandage circulaire mais bien de feuilles laminées pour attelles et orthèses, l'opérateur doit tenir compte du retrait atteignant parfois 10 pour cent, qui se produit lors du chauffage préparatoire de la feuille, ce qui est gênant. Il y a là aussi intérêt à pouvoir utiliser des substrats textiles moins déformables.

Dès lors que les applications des produits visés par la présente invention comprennent un cycle de chauffage et de refroidissement, il est utile de définir au moins quatre températures caractéristiques dont il est intéressant d'examiner aussi les valeurs pour apprécier les avantages procurés par l'invention au-delà du simple contrôle de la fluidité et de la thermoadhésivité.

La température (T1) de ramollissement est la température à laquelle le matériau commence à perdre sa rigidité. Elle peut être déterminée notamment par méthode ASTM 1043-87 ou 1053-89 en prenant le point d'inflexion de la courbe de variation du module avec la température.

La température (T2) de traitement pour l'application est la température convenable à laquelle il faut porter le matériau pour que celui-ci acquière en un temps raisonnable les caractéristiques de formabilité et d'adhésivité recherchées et les conserve suffisamment pendant le temps nécessaire à l'achèvement de l'application. Il est à noter que pendant l'application, la matière refroidit de manière naturelle ou forcée. Suivant l'épaisseur de la matière et la température d'ambiance, cette température T2 sera plus ou moins élevée, nécessairement au-delà de la température thermodynamique de fusion du constituant semi-cristallin dans la combinaison.

La température (T3) est la température à laquelle, lors du refroidissement de la matière, normalement après la fin de l'application, la matière commence à perdre son pouvoir adhésif.

La température (T4) est la température à laquelle lors du refroidisssement de la matière, normalement après la fin de l'application, la matière commence à perdre son aptitude à être formée par accroissement de sa rigidité.

Dans la pratique, on fait plus souvent référence au temps (t3) après lequel la matière n'est plus adhésive et au temps (t4) après lequel la matière n'est plus déformable lorsqu'elle refroidit en épaisseur donnée, dans une ambiance donnée et à partir d'une température de traitement donnée. Dans ces conditions, il y a correspondance entre t3 et T3 ainsi qu'entre t4 et T4.

Cette distinction des quatre températures ci-dessus permet de préciser les caractéristiques optimales des produits visés, de ce point de vue.

D'une manière générale, il faut que la température T1 soit la plus élevée possible au-delà de la température ambiante, que la température T2 soit la moins élevée possible pour permettre une application aisée sans risque de brûlures, ce qui est particulièrement important dans le cas du bandage orthopédique.Il faut que la température T3 soit la plus basse possible pour donner un délai maximum t3 à l'opérateur chargé de l'application. La valeur optimale de T4 est fonction de l'application et du temps t4 que l'opérateur souhaite se réserver pour achever son travail. Le temps t4 doit être suffisant mais pas au point que l'opérateur doive attendre pour juger de son résultat.

Signalons à propos de ces températures caractéristiques, que le brevet allemand DE-3605192 A1 suggère un moyen pour obtenir précisément une mise en forme d'une feuille thermoadhésive, qui n'est pas destinée à être laminée mais bien à assembler des éléments de chaussures à une température la plus en-deça possible de la température à laquelle la feuille devient adhésive, et ce par enduction partielle seulement d'un substrat, éventuellement imprégné d'un polymère, par un produit thermoadhésif. Il va de soi que ce savoir-faire qui vise dans notre nomenclature un écart maximum entre T1 et T2, n'est pas applicable dans les domaines visés par la présente invention, où on cherche au contraire une stabilité dimensionnelle du composite dans l'intervalle de température le plus large possible.

L'objet de la présente invention est de procurer des matières devenant facilement moulables à la main jusqu'à de grandes dimensions de un ou deux mètres carrés, à partir d'un traitement à une température la plus basse possible en dessous de 100°C, tout en gardant les propriétés de température ambiante jusqu'à une température la plus élevée possible au-dessus de la température ambiante.

Un autre objet de la présente invention est de procurer ces matières sous forme de bandages orthopédiques thermoplastiques, légers et aérés, présentant sans devoir nécessairement recourir à une pellicule d'un film intercalaire, une bonne résistance interlaminaire, une bonne rigidité et une bonne résistance à la déchirure, sans que la bande ne devienne une masse compacte ni transfère la résine qu'elle contient sur des corps autres qu'elle-même pendant son application à chaud.

Un autre objet de la présente invention est de procurer également ces matières sous forme de bandages souples ou semi-rigides qui peuvent être laminés par traitement à température et qui redeviennent secs a froids.

Un autre objet de la présente invention est de procurer ces composites sous forme de plaques et feuilles composites facilement moulables et adhérant sur elles-mêmes et qui gardent leur forme pendant le moulage sans risque de déformation incontrôlable due à une trop grande fluidité, de sorte qu'un seul opérateur peut préparer par laminage, une pièce de grande dimension comme un lombostat, un décor de théâtre ou l'empreinte d'un objet de plus d'un mètre de dimension moyenne.

Un autre objet de la présente invention est d'obtenir ces composites par des procédés économiques faisant intervenir le moins possible ou pas de solvant.

Un autre objet de la présente invention est de rendre ces matières recyclables et réutilisables.

Dans la présente invention, on a obtenu plusieurs manières de contrôler la fluidité à T2, l'adhésivité, la rigidité et les températures caractéristiques de composites thermoadhésifs sous forme de plaques, de feuilles ou encore de textiles à mailles ouvertes enduits de ces matières, formables et moulables dans les conditions décrites plus haut d'un travail aisé à une température qui ne gêne pas une application manuelle notamment.

Selon la présente invention, on a trouvé de manière inattendue que certaines combinaisons composites de structures polymères amorphes ou semi-cristallines qui ont un caractère élastovisqueux à càoutchoutique au-dessus d'une certaine température, avec une proportion variable suivant les cas, de structures polymères thermoplastiques essentiellement semi-cristallines et contenant une quantité minimum d'unités structurales de type ester aliphatique, permettent de pallier les difficultés citées plus haut d'une trop grande fluidité et d'une adhésivité excessive, tout en donnant des possibilités nouvelles de moduler ces caractéristiques ainsi que d'autres caractéristiques utiles dans l'application ou l'usagage des matières qui en découlent.

Dans la suite de cette description, on appellera respectivement, "premier constituant" et "second constituant", les deux constituants de la combinaison définis plus haut tels qu'appliqués successivement sur un substrat textile ou plastique.

La combinaison de la présente invention comprend un premier constituant polymérique amorphe ou semi-cristallin ayant dépassé son point de ramollissement thermique au moins à la température T2 et présentant à cette même température au moins, un comportement élastovisqueux à caoutchoutique, avec un second constituant essentiellement semi-cristallin d'un contenu en unités structurales de type ester aliphatique d'au moins 80 pour cent environ et présentant à la température T2, un comportement essentiellement plastique et telle que le premier constituant est appliqué en premier lieu sur le substrat du composite et le second constituant en dernier lieu, formant un film continu ou enrobant complètement les brins si le substrat est par exemple, un tricot à mailles ouvertes.

Les combinaisons telles que décrites ci-avant se sont avérées thermoadhésives de manière tout à fait satisfaisante sans présenter l'inconvénient d'une déformation incontrôlable pendant l'application. Par le choix de la proportion et des éléments du premier constituant, et aussi dans une certaine mesure par le choix des éléments du second constituant, on a remarqué qu'il était aussi possible d'adapter les températures caractéristiques, la souplesse pendant l'application et la rigidité du produit obtenu, selon les besoins.

Selon la présente invention, le second constituant est choisi d'une manière qu'il ait des propriétés thermoadhésives intrinsèques alors que le premier constituant n'en a pas nécessairement. Inversément on a trouvé que le premier constituant doit conférer à la combinaison un caractère élastovisqueux lorsque celle-ci est soumise à la température de traitement préparatoire, ce qui a été obtenu avec certains premiers constituants présentant eux-mêmes un caractère élastovisqueux à caoutchoutique, alors que le second constituant ne peut être que plastique à cette même température.

Ainsi, il a été surprenant de constater que des bandages sur base d'un tricot à mailles ouvertes d'abord imprégné d'un latex d'un copolymère à base d'esters acrylate, méthacrylate, vinylique ou d'autre polymère ou copolymère et ayant une température de ramolissement pouvant aller de -40°C à +60°C puis séché, ensuite enrobé d'une solution de polymère à base de polycaprolactone ou d'autres moyens donnant lieu à un polymère thermoplastique semi-critallin comprenant au moins 80 pour cent d'unités structurales de type ester aliphatique donnent après évaporation du solvant une bande que l'on peut bobiner facilement et qui, après immersion dans un bain d'eau à 70°C, peut être déroulée sans difficulté alors que la même bande imprégnée d'un poids total équivalent du même polycaprolactone devient une masse indivisible si on n'a pas placé une pellicule de polyéthylène intercalaire, ce qui est surprenant vu que la matière en contact à l'interface est alors identique dans les deux cas.

Dans ce mode d'exécution de l'invention, on a trouvé que la rigidité du bandage sec après sa réalisation, est directement fonction de la température de ramollissement du premier constituant, plus rigide qu'un bandage non composite si la température de ramollissement du premier constituant est supérieure à 15°C environ et moins rigide dans le cas contraire.

Dans un premier groupe de polymères permettant d'obtenir le premier constituant, la famille des polymères et copolymères vinyliques, d'ester acryliques ou méthacryliques s'est montrée particulièrement intéressante.

Dans cette présentation composite du matériau, le premier constituant de type vinylique, acrylique, méthacrylique ou diénique est appliqué par imprégnation ou enduction d'un substrat textile d'abord et recouvert ensuite par le second constituant. Suivant ce mode d'exécution de l'invention, un mode préféré est d'appliquer ce premier constituant sous forme de dispersion aqueuse telle qu'un latex du polymère ou du copolymère. On peut ainsi déposer une quantité importante de matière solide sans nuire à l'environnement. Le second constituant enrobant le premier peut être appliqué de diverses manières, par exemple, par dépôt d'une solution de polymère ou d'une solution de monomères pouvant donner lieu à la formation du polymère sur le substrat pendant ou après évaporation du solvant.

Ce mode d'exécution préféré de l'invention donne accès à une grande diversité de produits tels que des bandages thermoadhésifs qui restent souples et non collants à froid si on choisit pour le premier constituant un latex d'un polymère dont la température de transition vitreuse est inférieure à environ 15°C et notamment de l'ordre de moins 20°C, ou bien des produits de contention de grande dimension qui sont relativement peu déformables entre les températures T2 et T4 de sorte qu'ils gardent facilement la forme qui leur est donnée pendant le laps de temps t4 et qui sont de haute rigidité à froid, si à l'inverse, on choisit pour le premier constituant un latex d'un polymère dont la température de transition vitreuse se situe dans l'intervalle T2 à T4, soit par exemple à environ 40°C comme on peut l'obtenir par exemple en polymérisant par exemple de l'isobutylméthacrylate ou un mélange d'acrylate de méthyle et de méthacrylate d'ethyle en émulsion.

Les possibilités de combinaisons dans ce mode d'exécution de l'invention sont tellement nombreuses qu'il n'est pas possible de les exposer toutes. Elles permettent en particulier comme déjà signalé plus haut d'obtenir un produit pour lequel T3 est inférieure à T4 ce qui est inaccessible par le savoir-faire antérieur. On a pu obtenir cette caractéristique en appliquant une quantité de plus de 50 pour cent du premier constituant de type polyéthylméthacrylate en émulsion puis en enrobant ce dernier par le second constituant à base de polycaprolactone. Après un traitement à 75°C, on constate que le matériau est encore adhésif alors qu'il est redevenu pratiquement rigide.

Par ce mode d'exécution particulier de l'invention, on obtient des bandages notamment ou des produits composites pour attelles, orthèses ou autre application qui peuvent être soit plus souples, soit plus rigides que ceux permis par le savoir-faire antérieur et en ayant dans le dernier cas la faculté d'ajuster la souplesse du produit pendant qu'il est mis en oeuvre c'est-à-dire dans l'intervalle de temps t4.

Dans un deuxième groupe de polymères permettant de représenter le premier constituant , on a trouvé qu'il était possible d'obtenir les mêmes effets avec un copolymère comme par exemple de type polyuréthanne ou polyurée qui est de nature élastovisqueuse a caoutchoutique au moins à la température T2 et dans l'intervalle T2 à T4 voire en deça, selon que les caractéristiques de ce polyuréthanne gouvernent la température T4 ou que celle-ci est gouvernée par les caratéristiques du second constituant respectivement.

on obtient le même type d'effet qu'avec les premiers constituants de type vinylique ou (méth)acrylique.

On obtient assez facilement ce constituant élastovisqueux à caoutchoutique au moins à la température T2, par réaction d'un mélange de diol, triol et diisocyanate notamment, mais on peut l'obtenir également au départ de réactifs difonctionnels uniquement si notamment, l'un deux est de nature intrinsèquement caoutchoutique comme le polytétraméthylèneglycol ou un polypropylèneoxyde. On peut évidemment employer en même temps un triol et un diol caoutchoutique.

Ce second type de premier constituant de nature polyuréthanne, peut par ailleurs être obtenu amorphe ou semi-cristallin à température ambiante mais dans ce dernier cas, sa température de ramollissement doit être inférieure à la température T2 visée, soit en tout cas inférieure à environ 95 à 100 °C et de préférence inférieure à 80°C. Il va de soi que la réaction uréthanne n'est pas indispensable pour former ce type de premier constituant dans la combinaison intramoléculaire et que toute autre liaison chimique peut convenir.

Dans un mode d'exécution préféré de l'invention, suivant cette deuxième famille de premiers constituants, notamment si on cherche à obtenir une rigidité à température ambiante aussi élevée que possible, on choisit un polyuréthanne contenant au moins 50 pour cent environ d'unités structurales cristallisables et ayant une température de fusion inférieure à T2, telles que a base d'ester aliphatique d'une masse moléculaire d'au moins 2000 environ et de préférence supérieure à 3000.

Ces combinaisons apportent plusieurs avantages décisifs notamment par rapport aux seuls polymères d'ester cyclique et au polyuréthannes linéaires homopolymères du savoir-faire antérieur qui comme rappelé plus haut nécessitent de manière optimale le type de substrat à brins volumineux décrit ainsi qu'un film intercalaire pour la préparation des bobines de bandage orthopédique.

Il faut aussi remarquer dès maintenant que la présente invention élargit considérablement le spectre des applications qui en découlent. Elle permet notamment d'obtenir un bandage thermoadhésif souple qui prouve son utilité pour le traitement d'une luxation telle que la tendinite.

Dans cette application sous forme composite représentant le mode d'exécution de la présente invention, telle que bandages ou matériel de contention, au départ d'un substrat textile à mailles ouvertes notamment, on peut pour la deuxième famille de premiers constituants, aussi dissocier les deux constituants en une combinaison telle que le premier constituant de nature élastovisqueuse à caoutchoutique est déposé ou imprégné en premier lieu et le second constituant thermoadhésif est déposé en second lieu en un film continu ou enrobage total des brins d'un tricot à mailles ouvertes. On obtient un effet similaire et d'une même manière que lorsque le premier constituant est du type polyvinylique ou poly(méth)acrylate.

Pour les deux familles de premiers constituants, la quantité de second constituant par rapport à celle de premier doit alors être ajustée en fonction du caractère caoutchoutique du premier, de manière à assurer un joint adhésif suffisant. Si le premier constituant a des caractéristiques de caoutchouc relativement peu déformable, une quantité globale de deuxième constituant thermoadhésif supérieure à 50 pour cent peut être nécessaire car l'aire de contact possible au laminage est relativement faible. Si le premier constituant présente une plasticité suffisante entre T2 et T4, une quantité de deuxième constituant inférieure à 50 pour cent peut éventuellement suffire. Par un raisonnement géométrique simple, on comprend en effet aisément qu'une quantité de premier constituant de 49 pour cent par exemple donne par effet d'enrobage concentrique une épaisseur de second constituant qui n'est que de 30 pour cent du rayon et ce en faisant abstraction du volume occupé par le textile. Il n'est donc pas étonnant que cette technique demande néanmoins une proportion massique de constituant thermoadhésif relativement importante, son effet étant amoindri par la géométrie. Contrairement à ce à quoi on pourrait s'attendre toutefois, on a dû constater que les propriétés de rigidité du composite résultant, sont principalement dictées par les caractéristiques du premier constituant, ce qui est surprenant si on raisonne en termes de résistance des matériaux. En effet, rapporté à une masse unitaire, un tube creux est toujours plus rigide qu'un tube plein.

C'est donc l'effet composite après laminage qui gouverne cette rigidité. On a en effet obtenu par ce mode d'exécution de la présente invention un matériau composite dont la rigidité de chaque pli pendant la mise en oeuvre (entre T2 et T4) est inférieure à celle d'un pli équivalent et de composition homogène, alors que le composite résultant, à plusieurs plis, en a une rigidité supérieure, ce qui est surprenant.

D'une manière générale, les applications des produits de la présente invention sont beaucoup plus larges que celles du savoir-faire antérieur. En effet, on peut obtenir des matières qui permettent de réaliser facilement, à la main, des structures telles que bandages circulaires, orthèses et prothèses externes et de maintien mais aussi des bandages thermoadhésifs souples qui n'étaient pas connus, de même que des éléments de structure décorative de grande dimension.

Les bandages orthopédiques thermoplastiques notamment peuvent être fabriqués directement en largeur utile de 5, 10 et 15 cm par exemple et être bordés de lisières uniformes et lisses. Les attelles et orthèses thermoplastiques obtenues par imprégnation de tissu peuvent être suivant la présente invention, dimensionnellement stables car celle-ci permet d'utiliser des tricots à brins de fibres continues, relativement serrés et résitant à l'allongement pendant l'étape d'imprégnation. D'une autre manière, ce mode d'exécution de la présente invention, permet si on le désire, de renforcer un tissu plus déformable en l'imprégnant d'abord d'un polymère tel que décrit, sous forme de latex par exemple.

On peut moduler les températures caractéristiques beaucoup plus largement que par le savoir-faire antérieur et obtenir des souplesses et rigidités variables du matériau aussi bien pendant sa mise à forme qu'après refroidissement.

### EXEMPLE

Sur un tricot de fibres polyester/coton à mailles ouvertes de 6mm de côté et d'un titre de 151 grammes par mètre carré, on a appliqué successivement par trempage des latex de copolymère acrylate/méthacrylate dont les températures de transition vitreuse sont respectivement de 11°C, 28°C et 41°C. Les bandes sechées, ont ensuite été enduites par imprégnation d'une solution dans le chlorure de méthylène d'une préparation comprenant 483.8 gr de polycaprolactone diol de masse moléculaire 4280, 3.4 grammes de polycaprolactone triol de masse équivalente 184, 19.7 grammes d'hexaméthylène-diisocyanate, environ 300 grammes de solvant et 0.15 ml de dibutyldilaurate d'étain. Après traitment au four à 95°C, on obtient des bandes dont la proportion des constituants est donnée au tableau ci-après en regard de la température de transition du latex et du module apparent obtenu après laminage de 3 couches de la bande à une température de 65°C.

**Tableau**

| Proportion en poids et rigidité en flexion suivant la température de ramollissement de la première couche. | | | |
|---|---|---|---|
| Température de ramollissement | 11°C | 28°C | 41°C |
| Fraction en poids tricot | 0.17 | 0.17 | 0.17 |
| Fraction en poids première couche | 0.24 | 0.29 | 0.29 |
| Fraction en poids seconde couche | 0.59 | 0.54 | 0.54 |
| Module apparent en flexion (3 plis laminés) (MPa) | 156 | 242 | 380 |

Les trois types de bandages bobinés en roulettes de 2 mètres de longueur et sans film intercalaire se prêtent bien à une application sur un membre lorsqu'elles ont été traitées à 60°C dans l'eau pendant une minute, et forment après 5 minutes environ un bandage, rigide ou souple suivant que la température de ramollissement du premier constituant est de 41°C ou 11°C.

## Revendications

1. Combinaison composite de matières en feuilles, en bandes roulées ou en plaques présentant des ouvertures uniformément réparties d'une dimension allant de 1 à 12 mm, laissant un passage libre d'au moins 40 % de la surface totale, formables et adhésives sur elles-mêmes à une température dans l'intervalle de températures de 35 à 90 °C, et associant un substrat textile à mailles ouvertes d'un poids spécifique n'excédant pas 500 g / m², dont le corps est successivement imprégné ou enduit, puis imprégné à nouveau et complètement enrobé de deux matériaux distincts au moins, dont le premier à l'intérieur ou en surface du substrat est de la famille des polymères, copolymères ou alliages amorphes ou semi-cristallins ayant une température de ramollissement n'excédant pas 80 °C et présentant au-delà de cette température de ramollissement un comportement élasto-visqueux à coutchoutique mais non fluide, et le dernier à la surface extérieure du premier, est de la famille des polymères, copolymères ou alliages semi-cristallins, d'un contenu en unités structurales de type ester aliphatique d'au moins 80 % et dont la température de fusion est comprise entre 35 et 80 °C, et ayant pendant un temps après la fusion un caractère adhésif sur soi-même de manière à pouvoir être laminée en deux ou plusieurs couches.

2. Combinaison selon la revendication 1, où le substrat est un tissu ou un substrat non tissé dont les ouvertures vont de 1 à 12 mm et où tous les matériaux sont appliqués sur le substrat avant perforation ou déploiement.

3. Combinaison selon la revendication 1, où le premier matériau est en proportion pondérale de 10 à 90 %, de préférence de 20 à 80 %, et le second de 90 à 10 %, de préférence de 80 à 20 %, entre eux, le poids du substrat allant de 5 à 50 % de l'ensemble de la combinaison.

4. Combinaison selon la revendication 1, où le premier matériau est choisi parmi le groupe constitué par un polymère ou copolymère vinylique, un polymère ou copolymère d'ester acrylique ou méthacrylique, un polymère ou copolymère de butadiène, chloroprène ou isoprène, un copolymère de styrène, d'acrylonitrile et de butadiène, de chloroprène or d'isoprène, un copolymère d'éthylène et d'ester acrylique, méthacrylique ou vinylique, un polyuréthanne thermoplastique, un polyuréthanne ou un polyurée.

5. Combinaison selon la revendication 1, ou le premier matériau est un polymère branché ou partiellement ou totalement réticulé et contient en proportion de plus de 50 % l'unité structurale de polyesters aliphatiques cristallisables.

6. Procédé permettant d'obtenir la combinaison selon la revendication 4 ou 5, où le premier matériau est en tout ou en partie appliqué au substrat par dépôt ou imprégnation avec ou sans solvant d'une composition réactive de monomères ou macromères, lesquels se transforment dans ledit matériau en présence du substrat, lequel solvant éventuel est évaporé.

7. Procédé permettant d'obtenir la combinaison selon la revendication 4 ou 5, où le premier matériau est en tout ou en partie appliqué au substrat par dépôt ou imprégnation d'un latex de polymères suivi d'une évaporation de l'eau à une température qui permet la coalescence des particules de polymères.

8. Matériau de structure thermoadhésif et thermomoulable par laminage successif d'une composition selon l'une quelconque des revendications 1 à 5.

9. Elément de décoration en relief à base de matériau selon la revendication 8.

10. Elément laminé de coffrage perdu pour moulage de résines polyesters renforcées à base de matériau selon l'une quelconque des revendications 1 à 5.

11. Bandage rigide ou souple fait par laminage circulaire après traitement à température d'un matériau en bande selon l'une quelconque des revendications 1 à 5.

12. Elément de maintien ou de contention obtenu par laminage à température d'un matériau en feuille ou en plaque selon l'une quelconque des revendications 1 à 5.

## Claims

1. A composite combination of materials in sheets, wound strips or plaques, offering uniformly distributed openings of size from 1 to 12 mm, leaving a free passage of at least 40 per-cent of the total surface, formable and adhesive to themselves at a temperature in the temperature interval from 35°C to 90°C, and combining an open mesh textile substrate with specific weight not exceeding 500 g per square meter, whose body is successively impregnated or coated, then impregnated again and completely covered with at least two distinct materials, the first one, inside or on the surface of the substrate being of the group of amorphous or semi crystalline polymers, copolymers or alloys having a softening temperature not exceeding 80°C and showing above that softening temperature a viscoelastic to rubbery but non fluid behaviour, and the last one at the outer surface of the first ones, being of the group of semi crystalline polymers, copolymers or alloys with a content of aliphatic ester type structural units of at least !à per-cent and having a melting temperature comprised between 35 and 80°C and having during a certain time after the fusion an adhesive character on itself in a way the combination can be laminated in 2 or more layers.

2. A combination according to claim 1, wherein the substrate is a woven or non woven fabric with openings from 1 to 12 mm and wherein all the materials are applied prior to perforation or expansion.

3. The combination according to claim 1 wherein the first material is in weight proportion of 10 to 90 percent, preferably of 20 to 80 percent and the second in weight proportion of 90 to 10 percent, preferably of 80 to 20 percent, among themselves, the substrate weight being from 5 to 50 percent that of the whole combination.

4. A combination according to claim 1 wherein the first material is selected among the group a vinyl polymer or copolymer, a polymer or copolymer of acrylic or methacrylic ester, a polymer or copolymer of butadiene, chloroprene or isoprene, a copolymer of styrene, acrylonitrile and butadiene, chloroprene or isoprene, a copolymer of ethylene and acrylic, methacrylic or vinyl ester, a thermoplastic polyurethane, a polyurethane or a polyurea.

5. A combination according to claim 1 wherein the first material is a branched or partially or totally cross linked polymer and comprises in proportion larger than 50 percent the structural unit of crystallizable aliphatic polyesters.

6. A process allowing to obtain the combination according to claim 4 or 5 wherein the first material is in whole or in part applied onto the substrate by laying or impregnation with or without a solvent, of a monomer or macromer reactive composition which is transformed in said material in the presence of the substrate, said eventual solvent is evaporated.

7. A process allowing to obtain the combination according to claim 4 or 5 wherein the first material is in whole or in part applied onto the substrate by laying or impregnation of a polymer latex followed by the evaporation of the water at a temperature that allows the coalescence of the polymer particles.

8. A thermoadhesive and thermomouldable structural material for successive lamination having a composition according to one of claims 1 to 5.

9. A three dimensional decorative element based on material according to claim 8.

10. A laminated part for single use framing in the moulding of reinforced polyester resins, based on material according to one of claims 1 to 5.

11. A rigid or flexible bandage made by circular lamination after temperature treatment of a strip material according to one of claims 1 to 5.

12. A supporting or immobilisation device obtained by lamination at temperature of a sheet or plaque material according to one of claims 1 to 5.

## Patentansprüche

1. Verbund-Stoffverbindung in Form von Folien, aufgewickelten Bändern, oder Platten, die gleichmäßig verteilte Öffnungen mit einer Abmessung von 1 bis 12 mm aufweisen, einen freien Durchgang von mindestens 40 % der gesamten Oberfläche lassen, bei einer Temperatur in dem Temperaturbereich von 35 bis 90°C formbar sind und auf sich selbst kleben, und mit einem weitmaschigen Textilsubstrat mit einem spezifischen Gewicht von nicht mehr als 500 g/m² kombiniert sind, dessen Körper nacheinander mit mindestens zwei verschiedenen Materialien getränkt oder überzogen und dann erneut getränkt und vollständig umhüllt wird, wobei das erste Material, das sich im Inneren oder auf der Oberfläche des Substrats befindet, von der Familie der Polymere, Copolymere, oder amorphen oder halbkristallinen Legierungen ist, die eine Erweichungstemperatur von nicht mehr als 80°C haben, und jenseits dieser Erweichungstemperatur ein elastoviskoses bis gummiartiges, aber nicht flüssiges Verhalten aufweisen, und das zweite Material, das sich auf der äußeren Oberfläche des ersten Materials befindet, von der Familie der Polymere, Copolymere oder halbkristallinen Legierungen ist, die einen Gehalt an strukturellen Einheiten vom Typ aliphatischer Ester von mindestens 80 % haben, und deren Schmelztemperatur zwischen 35 und 80°C liegt, und die während einer gewissen Zeit nach der Schmelzung auf sich selbst kleben, so daß sie in zwei oder mehreren Schichten laminiert werden können.

2. Verbund-Stoffverbindung gemäß Anspruch 1, wobei das Substrat ein Gewebe oder ein nichtgewebtes Substrat ist, dessen Öffnungen von 1 bis 12 mm reichen, und bei dem alle Materialien vor der Perforation oder der Entfaltung auf das Substrat aufgebracht werden.

3. Verbund-Stoffverbindung gemäß Anspruch 1, wobei, bezogen auf das Gesamtgewicht beider Materialien, der Anteil des ersten Materials 10 bis 90 %, vorzugsweise 20 bis 80 %, und der Anteil des zweiten Materials 90 bis 10 %, vorzugsweise 80 bis 20 % beträgt, und das Gewicht des Substrats zwischen 5 und 50 % des Gewichts der gesamten Verbund-Stoffverbindung liegt.

4. Verbund-Stoffverbindung gemäß Anspruch 1, wobei das erste Material innerhalb der Gruppe gewählt wird, die gebildet wird von einem Vinylpolymer oder Vinylcopolymer, einem Polymer oder Copolymer von Acrylester oder Methacrylester, einem Polymer oder Copolymer von Butadien, Chloropren oder Isopren, einem Copolymer von Styrol, Acrylnitril und Butadien, Chloropren oder Isopren, einem Copolymer von Äthylen und Acrylester, Methacrylester oder Vinylester, einem thermoplastischen Polyurethan, einem Polyurethan oder einem Polyharnstoff.

5. Verbund-Stoffverbindung gemäß Anspruch 1, wobei das erste Material ein verzweigtes oder teilweise oder vollständig vernetztes Polymer ist, und die strukturelle Einheit aus kristallisierbaren, aliphatischen Polyestern in einem Anteil von mehr als 50 % enthält.

6. Verfahren, das ermöglicht, die Verbund-Stoffverbindung gemäß dem Anspruch 4 oder 5 zu erhalten, wobei das erste Material ganz oder teilweise als reaktive Zusammensetzung von Monomeren oder Makromeren durch Ablagerung oder Tränkung, mit oder ohne Lösungsmittel, auf das Substrat aufgebracht wird, wobei sich die Monomere oder Makromere in Gegenwart des Substrats in das Material umwandeln, und das eventuelle Lösungsmittel verdampft wird.

7. Verfahren, das ermöglicht, die Verbund-Stoffverbindung gemäß dem Anspruch 4 oder 5 zu erhalten, wobei das erste Material ganz oder teilweise als ein Latex aus Polymeren durch Ablagerung oder Tränkung auf das Substrat aufgebracht wird, wonach das Wasser bei einer Temperatur verdampft wird, die die Koaleszenz der Polymerpartikel ermöglicht.

8. Material von thermoklebender und thermopreßformoarer Struktur, erhalten durch aufeinanderfolgende Laminierung einer Verbund-Stoffverbindung gemäß irgendeinem der Ansprüche 1 bis 5.

9. Plastisches Dekorationselement auf der Basis von Material gemäß dem Anspruch 8.

10. Verlorenes, laminiertes Verschalungselement zum Preßformen von verstärkten Polyesterharzen auf der Basis von Material gemäß irgendeinem der Ansprüche 1 bis 5.

11. Starre oder flexible Bandage, hergestellt durch kreisförmige Laminierung, nach Warmbearbeitung, eines bandförmigen Materials gemäß irgendeinem der Ansprüche 1 bis 5.

12. Halte- oder Zurückhalteelement, erhalten durch Thermolaminierung eines folien- oder plattenförmigen Materials gemäß irgendeinem der Ansprüche 1 bis 5.
